# NEW EUROPEAN PATENT SPECIFICATION

(11) **EP 0 626 861 B2**
(45) Date of publication and mention of the opposition decision: **28.04.2004**
(45) Mention of the grant of the patent: 19.03.1997
(21) Application number: 93902914.6
(22) Date of filing: 12.01.1993
(51) Int. Cl.: A61K 39/395

(54) **TREATMENT FOR ASTHMA**
BEHANDLUNG VON ASTHMA
TRAITEMENT DE L'ASTHME

(30) Priority: 13.01.1992 US 821768
(43) Date of publication of application: 07.12.1994
(73) Proprietor: BIOGEN, INC., Cambridge Massachusetts 02142 (US)
(72) Inventor: LOBB, Roy, R., Westwood, MA 02090 (US)
(74) Representative: Wichmann, Hendrik, Dr.
(86) International application number: PCT/US1993/000030
(87) International publication number: WO 1993/013798

(56) References cited:
- EP-A1- 0 239 400
- EP-B1- 0 125 023
- WO-A1-91/03252
- CHEMICAL ABSTRACTS, vol. 115, no. 23, 09 December 1991, Columbus, OH (US); S. PARMENTIER et al., p. 539, no. 252791h/
- CHEMICAL ABSTRACTS, vol. 114, no. 9, 04 March 1991, Columbus, OH (US); R. ONISH et al., p. 545, no. 79730s/
- CHEMICAL ABSTRACTS, vol. 106, no. 5, 02 February 1987, Columbus, OH (US); H.G. BLUESTEIN et al., p. 395, no. 31234r/
- ELWOOD et al., Am. Rev. Respir.Dis. 145, 1289-1294 (1992)
- VRIEND, G., J. Mol. Graphics 8, 52-56 (1990)
- PADLAN, E.D., Molecular Immunology 31, 169-217 (1994)
- LEE et al., Science 237, 175-178 (1987)
- MOULD et al., J. Biol. Chem. 271, 20365-20374
- WELLS, J.A. and Lowman, Current Opinion in Structural Biology 2, 355-362 (1992)
- LYTTLE, M.H., Drug Dev.Res. 35, 230-236 (1995)
- CLASSEN/DIEHL/KOCHSICK, Innere Medizin, 1991, p. 1101-1104
- WEGENER et al., Science 274, 456-459 (1990)
- OSBORN, L., Cell 62, 3-6 (1990)
- MERCK Manual of Diagnosis and Therapy, 15th Edition, 623-634 (1987)

### SEQUENCE LISTING

(1) GENERAL INFORMATION:
   (i) APPLICANT:
      (A) NAME: Biogen, Inc.
      (B) STREET: 14 Cambridge Center
      (C) CITY: Cambridge
      (D) STATE: Mass
      (E) COUNTRY: USA
      (F) POSTAL CODE (ZIP): 02142

      (A) NAME: Roy R. Lobb
      (B) STREET: 62 Loring Street
      (C) CITY: Westwood
      (D) STATE: Mass
      (E) COUNTRY: USA
      (F) POSTAL CODE (ZIP): 02090
   (ii) TITLE OF INVENTION: Treatment for Asthma
   (iii) NUMBER OF SEQUENCES: 4
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (v) CURRENT APPLICATION DATA:
      APPLICATION NUMBER: PCT/US93/00030
   (vi) PRIOR APPLICATION DATA:
      (A) APPLICATION NUMBER: US 821768
      (B) FILING DATE: 13-JAN-1992
(2) INFORMATION FOR SEQ ID NO:1:
   (i) SEQUENCE CHARACTERISTICS:
      (A) LENGTH: 360 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: misc_feature
      (B) LOCATION: 1
      (D) OTHER INFORMATION: /note- "pBAG159 insert: HP1/2 heavy chain variable region; amino acid 1 is Glu (E) but Gln (Q) may be substituted"
   (ix) FEATURE:
      (A) NAME/KEY: CDS
      (B) LOCATION: 1..360
   (xi) SEQUENCE DESCRIPTION: SEQ ID NO:1:
(2) INFORMATION FOR SEQ ID NO:2:
   (i) SEQUENCE CHARACTERISTICS:
      (A) LENGTH: 120 amino acids
      (B) TYPE: amino acid
      (D) TOPOLOGY: linear
   (ii) MOLECULE TYPE: protein
   (xi) SEQUENCE DESCRIPTION: SEQ ID NO:2:
(2) INFORMATION FOR SEQ ID NO:3:
   (i) SEQUENCE CHARACTERISTICS:
      (A) LENGTH: 318 base pairs
      (B) TYPE: nucleic acid
      (C) STRANDEDNESS: double
      (D) TOPOLOGY: linear
   (ii) MOLECULE TYPE: cDNA
   (ix) FEATURE:
      (A) NAME/KEY: CDS
      (B) LOCATION: 1..318
      (D) OTHER INFORMATION: /product- "HP1/2 light chain variable region"
   (ix) FEATURE:
      (A) NAME/KEY: misc_feature
      (B) LOCATION: 1
      (D) OTHER INFORMATION: /note- "pBAG172 insert: HP1/2 light chain variable region"
   (xi) SEQUENCE DESCRIPTION: SEQ ID NO:3:
   AGT ATT GTG ATG ACC CAG ACT CCC AAA TTC CTG CTT GTT TCA GCA GGA      48
   Ser Ile Val Met Thr Gln Thr Pro Lys Phe Leu Leu Val Ser Ala Gly
    1               5                  10                  15
   GAC AGG GTT ACC ATA ACC TGC AAG GCC AGT CAG AGT GTG ACT AAT GAT      96
   Asp Arg Val Thr Ile Thr Cys Lys Ala Ser Gln Ser Val Thr Asn Asp
               20                  25                  30
   GTA GCT TGG TAC CAA CAG AAG CCA GGG CAG TCT CCT AAA CTG CTG ATA     144
   Val Ala Trp Tyr Gln Gln Lys Pro Gly Gln Ser Pro Lys Leu Leu Ile
           35                  40                  45
   TAT TAT GCA TCC AAT CGC TAC ACT GGA GTC CCT GAT CGC TTC ACT GGC     192
   Tyr Tyr Ala Ser Asn Arg Tyr Thr Gly Val Pro Asp Arg Phe Thr Gly
       50                  55                  60
   AGT GGA TAT GGG ACG GAT TTC ACT TTC ACC ATC AGC ACT GTG CAG GCT     240
   Ser Gly Tyr Gly Thr Asp Phe Thr Phe Thr Ile Ser Thr Val Gln Ala
   65                  70                  75                  80
   GAA GAC CTG GCA GTT TAT TTC TGT CAG CAG GAT TAT AGC TCT CCG TAC     288
   Glu Asp Leu Ala Val Tyr Phe Cys Gln Gln Asp Tyr Ser Ser Pro Tyr
                   85                  90                  95
   ACG TTC GGA GGG GGG ACC AAG CTG GAG ATC                             318
   Thr Phe Gly Gly Gly Thr Lys Leu Glu Ile
               100                 105
(2) INFORMATION FOR SEQ ID NO:4:
   (i) SEQUENCE CHARACTERISTICS:
      (A) LENGTH: 106 amino acids
      (B) TYPE: amino acid
      (D) TOPOLOGY: linear
   (ii) MOLECULE TYPE: protein
   (xi) SEQUENCE DESCRIPTION: SEQ ID NO:4:
   Ser Ile Val Met Thr Gln Thr Pro Lys Phe Leu Leu Val Ser Ala Gly
    1               5                  10                  15
   Asp Arg Val Thr Ile Thr Cys Lys Ala Ser Gln Ser Val Thr Asn Asp
               20                  25                  30
   Val Ala Trp Tyr Gln Gln Lys Pro Gly Gln Ser Pro Lys Leu Leu Ile
           35                  40                  45
   Tyr Tyr Ala Ser Asn Arg Tyr Thr Gly Val Pro Asp Arg Phe Thr Gly
       50                  55                  60
   Ser Gly Tyr Gly Thr Asp Phe Thr Phe Thr Ile Ser Thr Val Gln Ala
   65 70 75 80
   Glu Asp Leu Ala Val Tyr Phe Cys Gln Gln Asp Tyr Ser Ser Pro Tyr
   85 90 95
   Thr Phe Gly Gly Gly Thr Lys Leu Glu Ile
   100 105

## Description

### FIELD OF THE INVENTION

The present invention relates to a treatment for allergic asthma. More particularly, this invention relates to the use of antibodies recognizing the B1 or B2 epitope of the α₄ subunit of Very Late Antigen-4 (VLA-4), a ligand on certain leukocytes for the endothelial cell receptor Vascular Cell Adhesion Molecule-1 (VCAM-1), in the treatment of allergic asthma.

### BACKGROUND OF THE INVENTION

Asthma is a condition of the respiratory tract characterized by widespread, reversible narrowing of the airways (bronchoconstriction) and increased sensitivity (hyperresponsiveness) of the airways to a variety of stimuli. The familiar symptomology of asthma, i.e., coughing, wheezing, chest tightness, dyspnea, is caused by airway smooth muscle contraction, increased bronchial mucus secretion, and inflammation. Though seldom fatal, asthma has been estimated to affect 10-20% of school-aged children around the world, and hospital admissions for asthma in children have increased dramatically in recent years, one survey for the United States indicating that hospital admissions for children under 15 with asthma increased by at least 145% between 1970 and 1984. (See, M.R. Sears, 1990 [1].) Overall, it is estimated that 10 million Americans (4% of the population) have asthma, and some $4 billion is spent in treatment per year. (L.K. Altman, 1991 [2]; C. Starr, 1991 [3].)

The causes of asthma are not completely understood, however the study of agents that trigger acute asthmatic episodes supports the theory that asthma is an immunological reaction by a subject in response to specific allergens of the subject's environment. These "triggers" exacerbate asthma by causing transient enhancement of airway hyperresponsiveness. Triggers that have been found to induce airway hyperresponsiveness include inhaled allergens, inhaled low molecular weight agents to which the subject has become sensitized (e.g., by occupational exposure), viral or mycoplasma respiratory infections, and oxidizing gases such as ozone and nitrogen dioxide. These "inducing" triggers can be distinguished from "inciting" triggers of bronchospastic episodes which include exercise, cold air, emotional stress, pharmacological triggers, inhaled irritants. The common feature of inducing triggers is that they are associated with airways inflammation; inciting triggers produce smooth muscle contractions (bronchospasms) which depend on the underlying degree of hyperresponsiveness, rather than increasing airways responsiveness themselves. (See, D.W. Cockcroft, 1990 [4].)

The recognition that airways inflammation is a cause of transient (acute) and also persistent airway hyperresponsiveness has had an impact on the treatment of asthma sufferers. Early treatments for asthma focused on bronchoconstriction and led to the development of many effective bronchodilator drugs. The most commonly prescribed were beta₂-adrenoceptor agonists (epinephrine, isoproterenol, albuterol, salmeterol, etc.), xanthines (caffeine, theophylline, etc.) and cholinoceptor antagonists (atropine, acetylcholine, etc.). More recently, however, anti-inflammatory drugs have begun to replace bronchodilators as first-line treatments for asthma. Commonly prescribed anti-inflammatory agents for asthma include disodium cromoglycate (DSCG), nedocromil sodium, antihistamines such as ketotifen, and corticosteroids such as prednisolone. (See, F.M.C. Cuss, 1990 [5] and P.M. O'Byrne, 1990 [6].)

The inflammatory response in asthma is typical for tissues covered by a mucosa and is characterized by vasodilation, plasma exudation, recruitment of inflammatory cells such as neutrophils, monocytes, macrophages, lymphocytes and eosinophils to the sites of inflammation, and release of inflammatory mediators by resident tissue cells (e.g., mast cells) or by migrating inflammatory cells. (J.C. Hogg, 1990 [7].) In allergen-induced asthma, sufferers often exhibit a dual response to exposure to an allergen --an "early phase" response beginning immediately after exposure and lasting until 1-2 hours after exposure, followed by a "late phase" response beginning about 3 hours after exposure and lasting sometimes until 8-10 hours or longer after exposure. (D.W. Cockroft, 1990 [4].) Late phase response in allergen-induced asthma and persistent hyperresponsiveness have been associated with the recruitment of leukocytes, and particularly eosinophils, to inflamed lung tissue. (W.M. Abraham et al., 1988 [8].) Eosinophils are known to release several inflammatory mediators, e.g., 15-HETE, leukotriene C₄, PAF, cationic proteins, eosinophil peroxidase. (K.F. Chung, 1990 [9].)

Many of the drugs used to treat asthma have been found to block or neutralize the effects of the release of inflammatory mediators which regulate the inflammatory response. For example, beta₂-adrenoceptor agonists and DSCG are potent stabilizers of mast cells, which are capable of releasing many mediators, including histamine, prostaglandins, leukotrienes, platelet activating factor (PAF), and chemotactic factors for neutrophils and eosinophils; corticosteroids. as another example, complex with steroid hormone receptors, which leads to the synthesis of proteins, such as lipocortins, that produce anti-inflammatory effects. (F.M.C. Cuss, 1990 [5].)

Although known asthma medications have some effect on leukocyte recruitment into the lung (W.M. Abraham et al., 1990 [8]), none of these drugs is effective to directly block migration of leukocytes into inflamed tissues.

Inflammatory leukocytes are recruited to sites of inflammation by cell adhesion molecules that are expressed on the surface of endothelial cells and which act as receptors for leukocyte surface proteins or protein complexes. Eosinophils have recently been found to participate in three distinct cell adhesion pathways to vascular endothelium, binding to cells expressing intercellular adhesion molecule-1 (ICAM-1), endothelial ceil adhesion molecule-1 (ELAM-1), and vascular cell adhesion molecule-1 (VCAM-1). (P.F. Weller et al., 1991 [10]; G.M. Walsh et al., 1991 [11]; B.S. Bochner et al., 1991 [12]; and A. Dobrina et al., 1991 [13].) VCAM1 binds to the α₄β₁ integrin, VLA-4, which is expressed on various lymphoid cells, including eosinophils (Weller et al., 1991 [10]; Elices et al. 1990 [14]). That eosinophils express VLA-4 differentiates them from other inflammatory cells such as neutrophils, which bind to ELAM-1 and ICAM-1 but not VCAM-1.

PCT/WO92/00751 published on 23 January 1992 describes a pharmaceutical composition for the prophylaxis or treatment of diseases involving the binding of eosinophils to cells expressing surface vascular cell adhesion molecules. The pharmaceutical composition comprises a VCAM, or an anti-VCAM antibody, and a pharmaceutically acceptable excipient or carrier. The document falls for consideration under Article 54 (3) EPC.

The VLA-4 mediated adhesion pathway was investigated in an asthma model to examine the possible role of VLA-4 in leukocyte recruitment to inflammed lung tissue. It has now been discovered that administering an anti-VLA-4 antibody that is capable of binding to the B1 or B2 epitope of the α₄ subunit inhibits both the late phase response and airway hyperresponsiveness in allergic sheep. Surprisingly, administration of such an anti-VLA-4 led to a reduction in the number of both neutrophils and eosinophils in the lung at 4 hours after allergen challenge, even though both cells have alternate adhesion pathways by which they can be recruited to lung tissues. Also surprisingly, inhibition of hyperresponsiveness in the treated sheep was observed which continued to 1 week, even though infiltration of leukocytes, including neutrophils and eosinophils, was not significantly reduced over time.

### SUMMARY OF THE INVENTION

The present invention is based on novel methods for the treatment of asthma and provides new pharmaceutical compositions useful in the treatment of asthma. In particular, the present invention provides the manufacture of a medicament for use in a method comprising the step of administering to an asthma sufferer an effective amount of an anti-VLA-4 antibody that is capable of binding to the B1 or B2 epitope of the α₄ subunit, such as monoclonal antibody HP1/2, as defined in the claims. The desired anti-VLA-4 antibody is advantageously administered in vivo to a patient with chronic allergen-induced asthma, and serves to inhibit late phase response to allergens and to attenuate airway hyperresponsiveness.

### BRIEF DESCRIPTION OF THE DRAWINGS

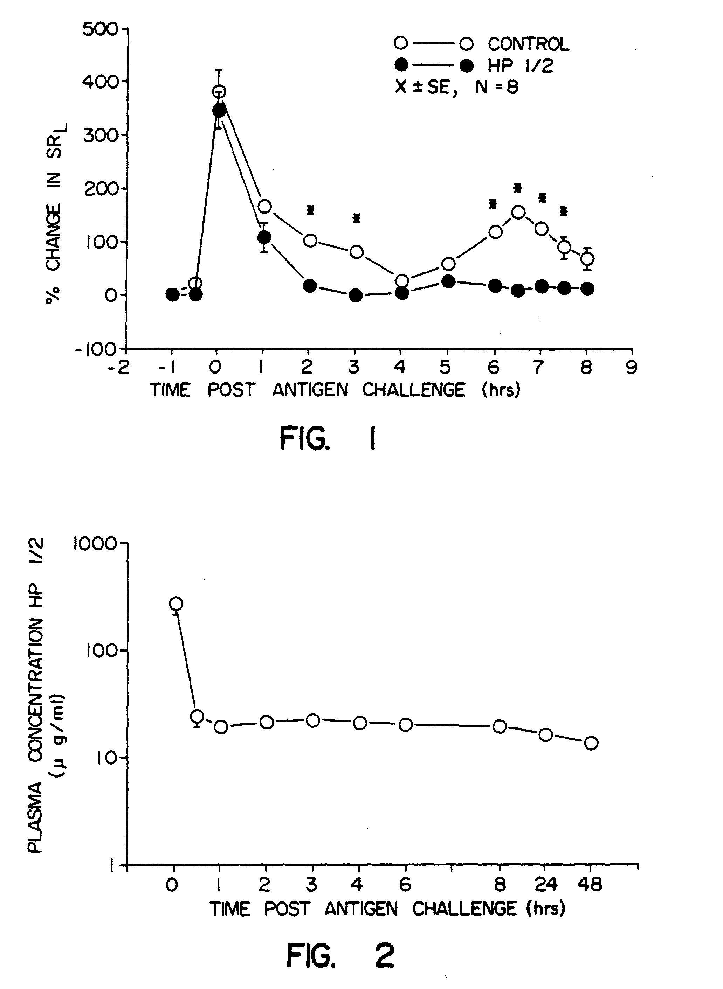

Figure 1 is a graph depicting the effect of monoclonal antibody HP1/2 (intravenous) on the response to allergen (Ascaris suum antigen) in dual responder allergic sheep. Percentage change in specific lung resistance (SR_{L}) is measured over time post allergen challenge. Asterisks indicate statistically significant results.
Figure 2 is a graph depicting plasma concentration of monoclonal antibody HP1/2 (intravenous) in sheep, measured over time after initial administration.

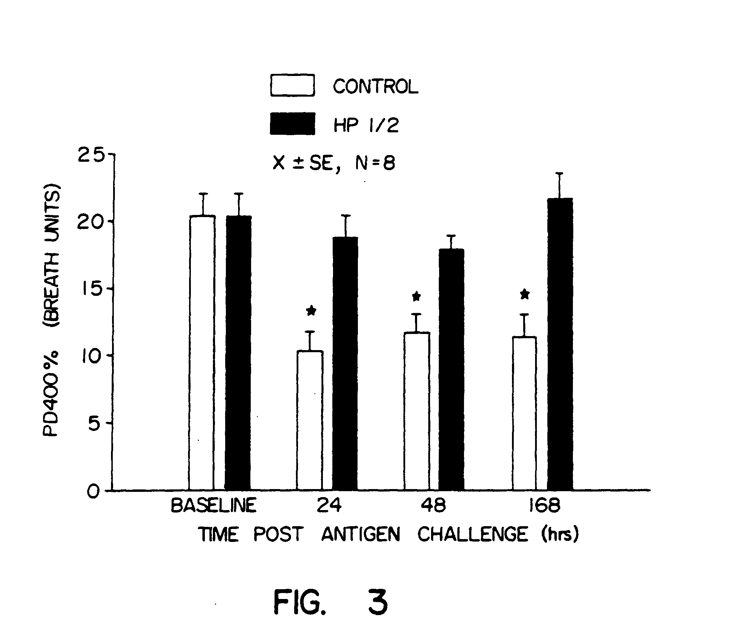

Figure 3 is a graph depicting the effect of monoclonal antibody HP1/2 (intravenous) on airway hyperresponsiveness in dual responder sheep. Airway responsiveness, measured in breath units (BU) of cumulative breaths of a 1% weight/volume carbachol solution (a known bronchoconstrictor) that increases specific lung resistance 400% over the value obtained using diluent alone. Asterisks indicate statistically significant results.

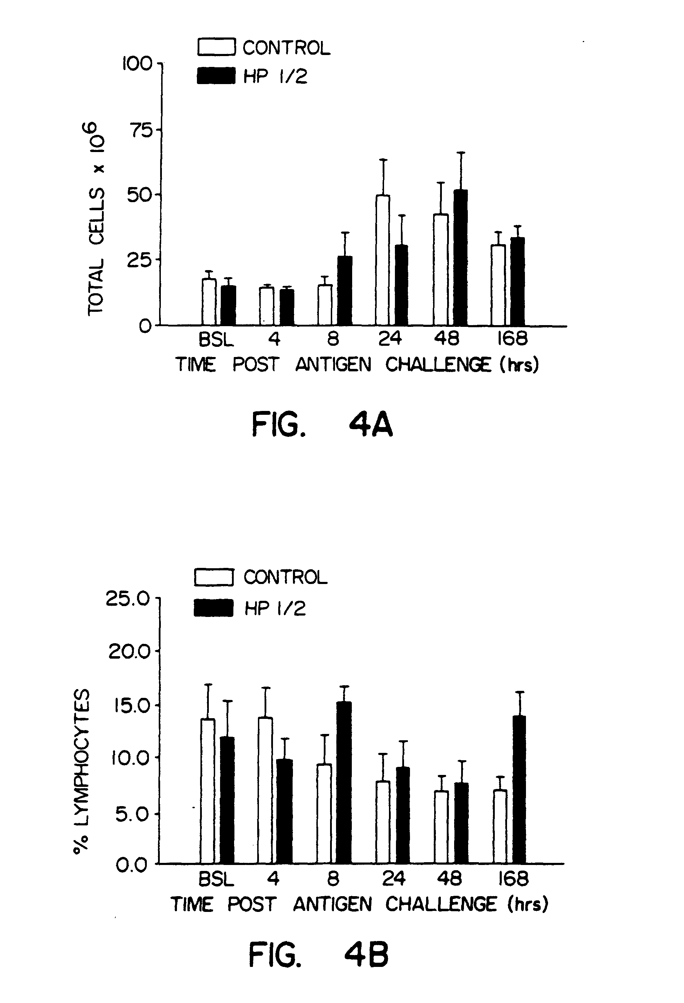

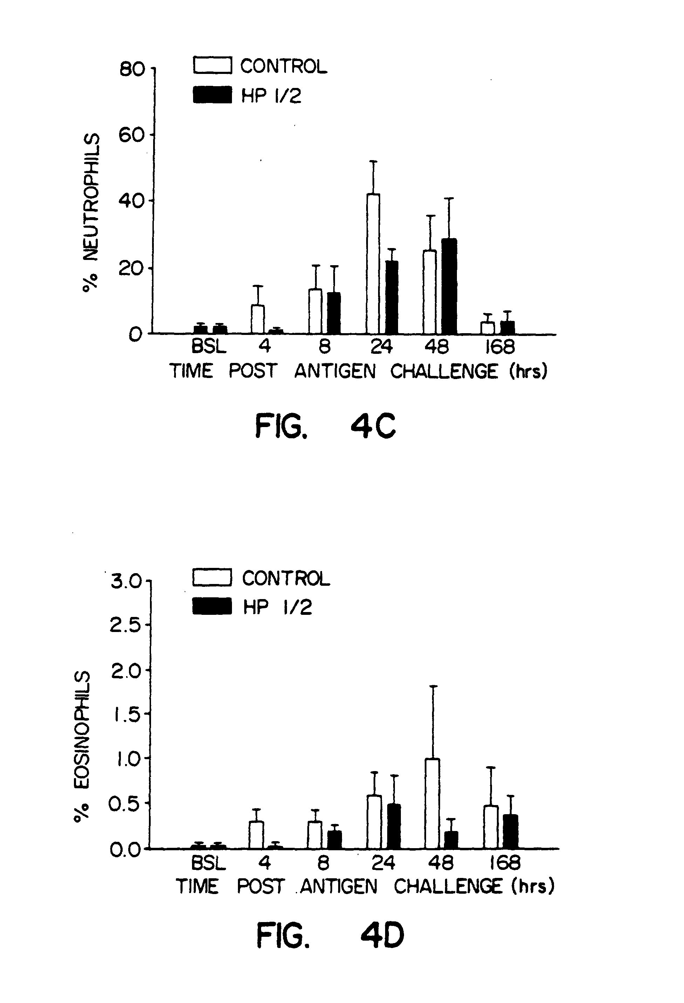

Figure 4 (comprised of Figures 4A, 4B, 4C and 4D) is a series of four graphs showing the total cells (Fig. 4A) and the levels of different leukocytes (lymphocytes (Fig. 4B), neutrophils (Fig. 4C), and eosinophils (Fig. 4D)) detected by bronchoalveolar lavage in allergic sheep challenged with Ascaris suum antigen alone and after pretreatment with monoclonal antibody HP1/2 (intravenous). Total cells, and the percentage of total cells that were lymphocytes or neutrophils or eosinophils, were measured at 4-hour, 8-hour, 24-hour, 48-hour and 1-week time points post allergen challenge.

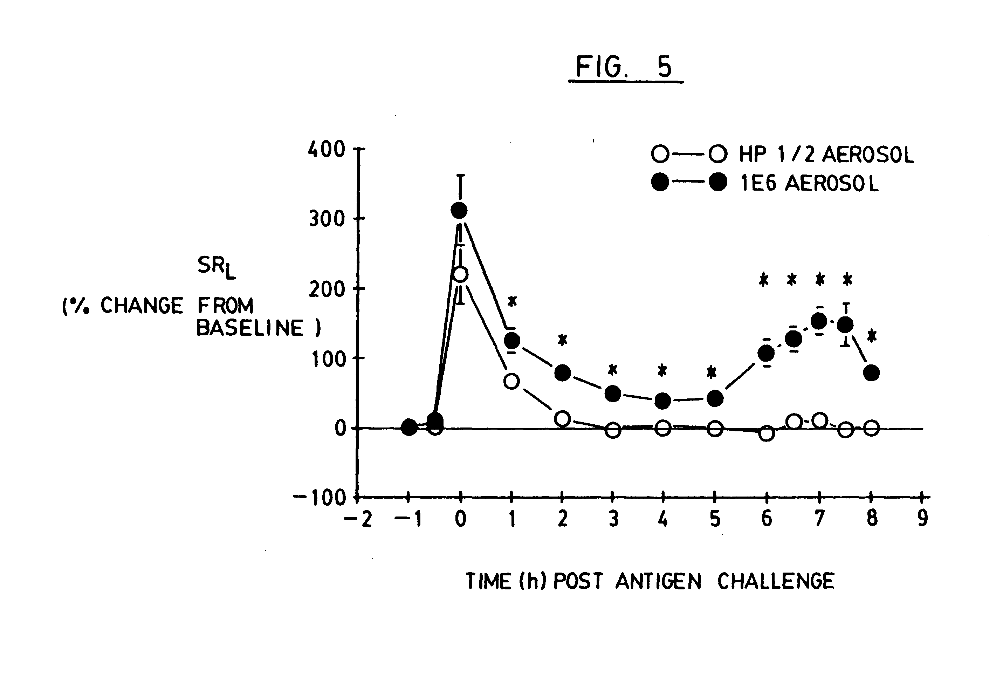

Figure 5 is a graph depicting the effect of monoclonal antibody HP1/2 (16 mg, aerosol) and 1E6 (16 mg, aerosol) on the response to allergen (Ascaris suum antigen) in dual responder allergic sheep. Percentage change in specific lung resistance (SR_{L}) is measured over time post allergen challenge. Asterisks indicate statistically significant results.

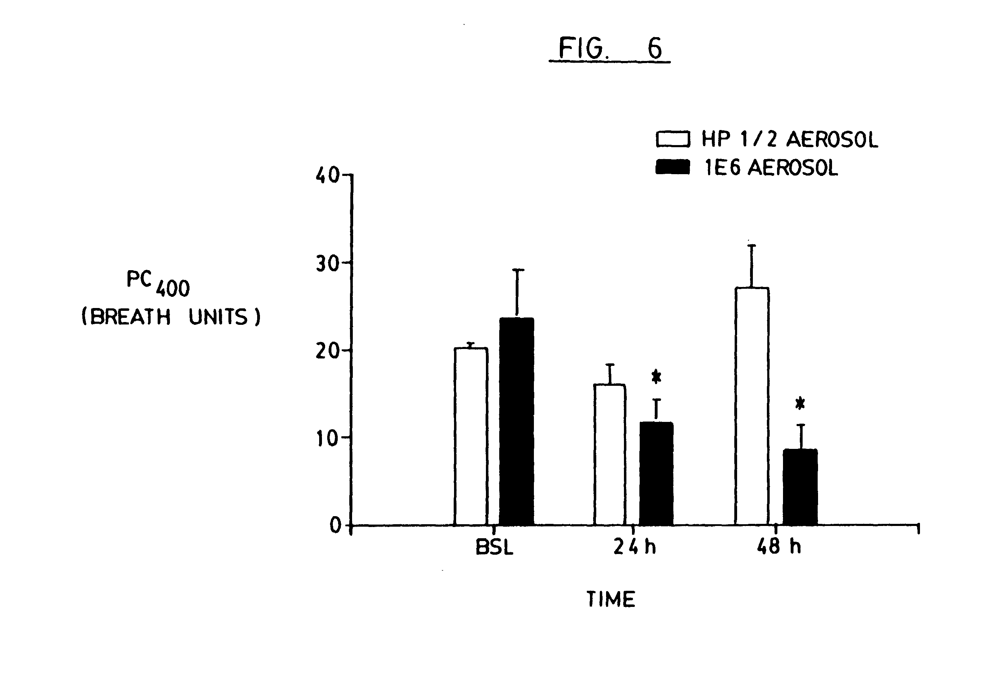

Figure 6 is a graph depicting the effect of monoclonal antibody HP1/2 (16 mg, aerosol) and 1E6 (16 mg, aerosol) on airway hyperresponsiveness in dual responder sheep. Airway responsiveness, measured in breath units (BU) of cumulative breaths of a 1% weight/volume carbachol solution (a known bronchoconstrictor) that increases specific lung resistance 400% over the value obtained using diluent alone. Asterisks indicate statistically significant results.

### DETAILED DESCRIPTION OF THE INVENTION

The technology for producing monoclonal antibodies is well known. Briefly, an immortal cell line (typically myeloma cells) is fused to lymphocytes (typically splenocytes) from a mammal immunized with whole cells expressing a given antigen, e.g., VLA-4, and the culture supernatants of the resulting hybridoma cells are screened for antibodies against the antigen. (See, generally, Kohler et al., 1975 [15].)

Immunization may be accomplished using standard procedures. The unit dose and immunization regimen depend on the species of mammal immunized, its immune status, the body weight of the mammal, etc. Typically, the immunized mammals are bled and the serum from each blood sample is assayed for particular antibodies using appropriate screening assays. For example, the desired anti-VLA-4 antibodies may be identified by immunoprecipitation of ¹²⁵I-labeled cell lysates from VLA-4-expressing cells. (See, Sanchez-Madrid et al. 1986 [16] and Hemler et al. 1987 [17].) The desired anti-VLA4 antibodies may also be identified by flow cytometry, e.g., by measuring flourescent staining of Ramos cells incubated with an antibody believed to recognize VLA-4 (see, Elices et al., (1990) [14]). The lymphocytes used in the production of hybridoma cells typically are isolated from immunized mammals whose sera have already tested positive for the presence of anti-VLA-4 antibodies using such screening assays.

Typically, the immortal cell line (e.g., a myeloma cell line) is derived from the same mammalian species as the lymphocytes. Preferred immortal cell lines are mouse myeloma cell lines that are sensitive to culture medium containing hypoxanthine, aminopterin and thymidine ("HAT medium").

Typically, HAT-sensitive mouse myeloma cells are fused to mouse splenocytes using 1500 molecular weight polyethylene glycol ("PEG 1500"). Hybridoma cells resulting from the fusion are then selected using HAT medium, which kills unfused and unproductively fused myeloma cells (unfused splenocytes die after several days because they are not transformed). Hybridomas producing a desired antibody are detected by screening the hybridoma culture supernatants. For example, hybridomas prepared to produce the desired anti-VLA-4 antibodies may be screened by testing the hybridoma culture supernatant for secreted antibodies having the ability to bind to the B1 or B2 epitope of a recombinant α₄-subunit-expressing cell line, such as transfected K-562 cells (see, Elices et al. [14]).

To produce the desired anti VLA-4-antibodies, hybridoma cells that tested positive in such screening assays were cultured in a nutrient medium under conditions and for a time sufficient to allow the hybridoma cells to secrete the monoclonal antibodies into the culture medium. Tissue culture techniques and culture media suitable for hybridoma cells are well known. The conditioned hybridoma culture supernatant may be collected and the desired anti-VLA-4 antibodies optionally further purified by well-known methods.

Alternatively, the desired antibody may be produced by injecting the hybridoma cells into the peritoneal cavity of an unimmunized mouse. The hybridoma cells proliferate in the peritoneal cavity, secreting the antibody which accumulates as ascites fluid. The antibody may be harvested by withdrawing the ascites fluid from the peritoneal cavity with a syringe.

Several anti-VLA-4 monoclonal antibodies have been previously described (see, e.g., Sanchez-Madrid et al., 1986 [16]; Hemler et al. (1987) [17]; Pulido et al. (1991) [19]). For the experiments herein, an anti-VLA-4 monoclonal antibody designated HP1/2 (obtained from Biogen, Inc., Cambridge, MA) was used. The variable regions of the heavy and light chains of the anti-VLA-4 antibody HP1/2 have been cloned, sequenced and expressed in combination with constant regions of human immunoglobulin heavy and light chains. Such a chimeric HP1/2 antibody is similar in specificity and potency to the murine HP1/2 antibody, and may be useful in the treatment according to the present invention. Similarly, humanized recombinant anti-VLA-4 antibodies may be useful therein. The HP1/2 V_{H} DNA sequence and its translated amino acid sequences are set forth in SEQ ID NO: 1 and SEQ ID NO: 2, respectively. The HP1/2 V_{K} DNA sequence and its translated amino acid sequence are set forth in SEQ ID NO: 3 and SEQ ID NO: 4, respectively.

Monoclonal antibodies such as HP1/2 and other anti-VLA-4 antibodies (e.g., Mab HP2/1, HP2/4, L25, P4C2) capable of recognizing the B1 or B2 epitopes of the VLA-α₄ chain (see, Pulido et al. (1991) [19]) will be useful in the present invention. While not wishing to be bound by one scientific theory, anti-VLA-4 antibodies used according to the present invention may specifically inhibit, at least for an initial period following allergen challenge, the migration of VLA-4-expressing leukocytes to inflamed sections of the lung. This inhibition of VLA-4 leukocyte migration could, in turn, prevent secondary pathological effects of leukocyte infiltration, e.g., release of toxic substances, inducement of soluble inflammatory cell mediators, release or inducement of leucocyte chemotactic agents (such as neutrophil chemotactic factors), etc. As a result late phase response to the allergen and continuing hypersensitivity of the airways may be attenuated. Alternatively, the desired anti-VLA-4 antibodies may attenuate signal transduction necessary for the release of inflammatory mediators and/or cell chemotactic agents.

The present invention provides the manufacture of a medicament for use in the treatment of asthma which comprises administering to a mammal suffering from allergic asthma a composition comprising the desired anti-VLA-4 antibody, as defined in the claims. The examples below set forth the results observed in asthmatic sheep. However, the similarity between physiological responses and pharmacological effects in sheep and in humans has been documented (see, e.g., W.M. Abraham, 1989 [20]); and similarities between sheep and other animal asthma models (rabbits, squirrel monkeys, guinea pigs, and sensitized dogs) have been noted (see, e.g., W.M. Abraham et al., 1988 [8]). Accordingly, the results reported herein will be relevant and applicable to, and the method claimed will be useful in, any mammal, including humans, suffering from allergic asthma.

The anti-VLA-4 antibody administered in accordance with the present invention may be administered prophylactically, before exposure to an asthma-inducing allergen. Beneficial effects will also be obtained if the antibody is administered at the time of or immediately after allergen exposure, between early phase and late phase response to attenuate the severity of late phase response, or at any time following allergen exposure to reduce or eliminate airway hyperresponsiveness.

The desired anti-VLA-4 antibody can be administered in the form of a composition comprising such an anti-VLA-4 antibody and a pharmaceutically acceptable carrier. Preferably, the composition will be in a form suitable for intravenous injection. Also contemplated are antibody compositions in the form of a sterile aqueous or phosphate-buffered saline solution which can be nebulized (atomized) and breathed directly into the lungs by the asthma sufferer, e.g., using an inhaler. Dosages will vary depending on the sensitivity of the asthma sufferer to particular allergens, the concentration of allergen on exposure and frequency/duration of exposure(s), the proposed mode of administration (e.g., injection or inhalation), the desired plasma level of antibody, the effectiveness of a particular antibody or combination of the desired VLA-4 antibodies in suppressing airway responsiveness, the clearance rate or half-life of the antibody composition, and other such factors familiar to physicians experienced in the treatment of allergic asthma. In general, dosages will be calculated and adjusted to maintain a plasma level of antibody in the range of from 1-1000 µg/ml, although higher or lower dosages may be indicated with consideration to the age, sensitivity, tolerance, and other characteristics of the patient, the acuteness of the flareup, the history and course of the disease, and other similar factors routinely considered by an attending physician. Depending on the potency and half-life of the antibody employed, it is preferred to use from about 0.05 mg/kg to 5.0 mg/kg of antibody, most preferably from 0.5 to 2.0 mg/kg of antibody, based on the weight of the patient receiving treatment.

Suitable pharmaceutical carriers include, e.g., sterile saline and physiological buffer solutions. Phosphate buffered saline (PBS) is preferred for inhalant administration. The pharmaceutical compositions may additionally be formulated to control the release of the active ingredients or to prolong their presence in the patient's system. Numerous suitable drug delivery systems are known for this purpose and include, e.g., hydrogels, hydroxmethylcellulose, microcapsules, liposomes, microemulsions, microspheres, and the like.

It will also be recognized that for the purposes of the present invention, antibodies capable of binding to the B1 or B2 epitope of the α₄ subunit of VLA-4 must be employed. It is preferred that monoclonal antibodies be used.

In addition to naturally produced antibodies, suitable recombinant antibodies capable of binding to the B1 or B2 epitope of the α₄ subunit of VLA-4 may alternatively be used. Such recombinant antibodies include antibodies produced via recombinant DNA techniques, e.g., by transforming a host cell with a suitable expression vector containing DNA encoding the light and heavy immunoglobulin chains of the desired antibody, and recombinant chimeric antibodies, wherein some or all of the hinge and constant regions of the heavy and/or the light chain of the desired anti-VLA-4 antibody have been substituted with corresponding regions of an immunoglobulin light or heavy chain of a different species (i.e., preferably the same spedes as the asthma sufferer being treated, to minimize immune response to the administered antibody). (See, e.g., P.T. Jones et al., 1986 [21], E.S. Ward et al., 1989 [22], and U.S. Patent 4,816,397 (Boss et al.) [23].)

Furthermore, the use of fragments of the desired anti-VLA-4 antibodies, such as Fab, Fab', F(ab')₂, and F(v) fragments; heavy chain monomers or dimers; light chain monomers or dimers; and dimers consisting of one heavy chain and one light chain are also contemplated herein. Such antibody fragments may be produced by chemical methods, e.g., by cleaving an intact antibody with a protease, such as pepsin or papain, or via recombinant DNA techniques, e.g., by using host cells transformed with truncated heavy and/or light chain genes. Heavy and light chain monomers may similarly be produced by treating an intact antibody with a reducing agent such as dithiothreitol or β-mercaptoethanol or by using host cells transformed with DNA encoding either the desired heavy chain or light chain or both.

When formulated in the appropriate vehicle, the pharmaceutical compositions contemplated herein may be administered by any suitable means such as orally, intraesophageally or intranasally, intrabronchially (local treatment, e.g., via bronchoscope), as well as subcutaneously, intramuscularly, intravenously, intra-arterially, or parenterally. Ordinarily administration via inhalation is preferred.

### EXAMPLES

Experiments were performed essentially as described by Abraham et al. [8]. Briefly, allergic sheep having natural allergic cutaneous reaction to 1:1000 or 1:10,000 dilutions of Ascaris suum extract (Greer Diagnostics, Lenoir NC) were tested, and sheep demonstrating both early and late phase airway response ("dual responders") to inhalation challenge with Ascaris suum antigen were selected. To measure breathing mechanics and physical changes in the airways, the sheep were restrained in a prone position with heads immobilized. A balloon catheter was advanced through one nostril under topical anesthesia with 2% lidocaine solution to the lower esophagus, and a cuffed endotracheal tube was advanced through the other nostril (using a flexible fiberoptic bronchoscope as a guide) for the measurement of airway mechanics and during aerosol challenges. Pleural pressure was estimated with the esophageal balloon catheter (filled with 1 ml of air) positioned 5-10 cm from the gastroesophageal junction. In this position, end expiratory pleural pressure ranged between -2 and -5 cm H₂O. Once the balloon was placed, it was secured so that it remained in position for the duration of the experiment. Lateral pressure in the trachea was measured with a sidehole catheter, (inner diam. 2.5 mm) advanced through and positioned distal to the tip of thee endotracheal tube. Transpulmonary pressure (the difference between tracheal and pleural pressure) was measured with a differential pressure transducer catheter system (MP45, Validyne, Northridge, CA). The pressure transducer catheter system showed no phase shift between pressure and flow to a frequency of 9 H_{Z}. Pulmonary resistance (R_{L}) was measured by connecting the proximal end of the endotracheal tube to a Fleich pneumotachograph (Dyna Sciences, Blue Bell PA). Signals indicating flow and transpulmonary pressure were recorded on an oscilloscope recorder (Model DR-12; Electronics for Medicine, White Plains, NY) linked to a computer for automatic calculation of pulmonary resistance (R_{L}) from transpulmonary pressure, respiratory volume (obtained by digital integration) and flow by the mid-volume technique, analyzed from 5-10 breaths. Thoracic gas volume (V_{tg}) was measured immediately after determination of R_{L} in a constant volume body plethysmograph. Specific lung resistance (SR_{L}) was calculated from these values (SR_{L} = V_{tg} x R_{L}).

Airway responsiveness was determined by performing dose response curves to inhaled carbachol. The dose response curves were plotted using measurements of SR_{L} taken immediately after inhalation of buffer (PBS) alone and after each consecutive administration of 10 breaths of increasing concentrations of carbachol in PBS. The concentrations of carbachol were 0.25%, 0.5%, 1.0%, 2.0% and 4.0% wt/vol in PBS. The provocation test was discontinued when SR_{L} increased over 400% from the post-PBS value or after the highest carbachol concentration had been administered. Airway responsiveness was determined by calculating from the dose response curves the cumulative carbachol dose in breath units (BU) that increased specific lung resistance 400% over the post buffer value (PD_{400%}). One breath unit was defined as one breath of a 1% wt/vol carbachol solution. Thus, the greater the suppression of airway hyper-responsiveness, the greater the number of breath units would be required before observing the same bronchoconstriction as seen in the controls.

Each sheep was subjected to a trial as a control in which a placebo (PBS without additive) was used as a pretreatment 30 minutes before allergen challenge with Ascaris suum antigen (Greer Diagnostics, Lenoir, NC). Subsequently, the sheep were subjected to an identical trial, except that 1 mg/kg of monoclonal antibody HP1/2 was administered to each sheep 30 minutes prior to antigen challenge. The placebo (buffer control or isotope-matched antibody (1 E6, anti-LFA3) control) and HP1/2 compositions were administered by intravenous injection. The HP1/2 composition (and the 1 E6 control) was prepared by diluting pure antibody obtained from a hybridoma (Biogen, Inc., Cambridge MA) in sterile, endotoxin-free PBS and adjusting to deliver 1 mg/kg antibody based on the weight of each sheep. The antigen solution was delivered as an aerosol using a disposable medical nebulizer (RAINDROP®, Puritan Bennett, Lenexa, KS) that provided an aerosol with a mass median aerodynamic diameter of 3.2 µM (geometric SD 1.9) as determined by an Andersen cascade impactor. The Ascaris suum extract was diluted in PBS to a concentration of 82,000 Protein Nitrogen Units(PNU)/ml. The output of the nebulizer was directed into a plastic T-tube, one end of which was connected to the inspiratory port of a Harvard respirator. A dosimeter connected to the nebulizer consisting of a solenoid valve and a 20 psi compressed air source and the solenoid valve was activated at the beginning of the inspiratory cycle of the Harvard respirator for one second. The aerosol delivered at a tidal volume of 500 ml and a rate of 20 breaths per min. for 20 min. Each sheep was challenged with an equivalent dose of antigen (400 breaths) in the control and HP1/2 trials. Carbachol aerosols for the dose response curves were also generated by nebulizer as described above.

For cellular analysis, bronchoalveolar lavage (BAL) was performed on each sheep. The distal tip of the specially designed 80 cm fiberoptic bronchoscope was gently wedged into a randomly selected subsegmental bronchus. Lung lavage was performed by slow infusion and gentle aspiration of 3 x 30 ml of PBS (pH 7.4) at 39° C, using 30 ml syringes attached to the working channel of the bronchoscope. The lavage return was collected, strained through gauze to remove mucus and then centrifuged at 420 g for 15 min. Supernatant was decanted, and the cells were resuspended in PBS. An aliquot of the suspension was transferred to a hemocytometer chamber to estimate total cells. Total viable cells were estimated by trypan blue exclusion. A second aliquot of the cell suspension was spun in a cytospin (600 rpm for 10 minutes) and stained by Wright-Giemsa and observed at 100X to identify cell populations. 500 cells per slide were characterized to establish the differential cell counts. Cells characterized included epithelial cells, macrophages, basophils, monocytes and unidentifiable cells (grouped into a category termed "others"), in addition to lymphocytes, neutrophils and eosinophils.

Plasma level of antibody and white blood cell counts were determined from venous blood samples (approx. 5 ml) from peripheral leg vein or jugular vein.

### Example 1

An airway challenge trial using eight dual responder allergic sheep was conducted according to the foregoing protocols. Baseline (BSL) airway responsiveness (PD_{400%}) was established 2-3 days prior to antigen challenge and a baseline bronchoalveolar lavage (BAL) was performed one day prior to challenge. On challenge day, baseline values for specific lung resistance (SR_{L}) was measured, then the sheep were administered buffer (control) or HP1/2 by injection. After this first administration ("treatment"), SR_{L} was measured, and 30 min. after treatment, the sheep were challenged with Ascaris suum antigen. SR_{L} was measured immediately after challenge, hourly from 1-6 hours following challenge, every half-hour from 6.5 hours to 8 hours, and also at 24 hours, 48 hours and 1 week (i.e., 168 hours) after antigen challenge. BALs were performed following SR_{L} measurements at 4, 8, 24 and 48 hours and at 1 week. For these studies, peripheral blood was drawn and total white blood cell counts and assessment of cell populations were taken before treatment (baseline), immediately after challenge, and at 1, 2, 3, 4, 6, 8, 24 and 48 hours, and 1 week after challenge. The results of this trial are shown in the figures:
Figure 1 shows the effect of HP1/2 treatment on antigen-induced airway responses in the subject sheep. HP1/2 treatment resulted in significant (indeed, virtually complete) inhibition of the late phase response experienced by the controls.
Figure 2 is a graph of plasma concentration of HP1/2 in µg/ml in the treated subjects, measured immediately following antigen challenge and then at 1, 2, 3, 4, 6, 8, 24 and 48 hours after challenge. After equilibration, the antibody concentration settled to a concentration of approximately 20 µg/ml, which concentration was maintained out to the 48-hour point.
Figure 3 is a graph showing the effect of HP1/2 treatment on airway responsiveness. At 24, 48, and 1 week after antigen challenge, treated subjects showed significant decrease in airway responsiveness. Even at 2 weeks after antigen challenge, treated subjects continued to show decreases in airway responsiveness. The fact that the virtually complete inhibitory effect of the antibody lasted out to 1 week is especially surprising and encouraging in terms of the therapeutic value of the treatment.
Figure 4 is a series of graphs illustrating the results of BALs performed at 4, 8, 24 and 48 hours after antigen challenge, and at 1 week after antigen challenge. The results show no significant changes over controls in total cells recovered from treated subjects. However, treated subjects showed reduced levels of both neutrophils and eosinophils at the 4-hour time point after challenge. This is somewhat surprising, given that the administration of anti-VLA-4 would not be expected to influence neutrophil recruitment, since neutrophils do not express VLA-4. Also, both neutrophils and eosinophils express alternative ligands involved in adhesion to endothelium; both types of cells have been shown to bind to endothelial cells via the LFA-1/ICAM-1 pathway and the CDX/ELAM-1 pathway.

Similar therapeutic effects with the anti-VLA-4 antibody HP1/2 were observed when the subjects were treated with HP1/2 antibody 2 hours after antigen challenge as opposed to 30 minutes prior to challenge as described above. The effect of HP1/2 was dose-dependent. For example, reducing the dose to 0.2 mg/kg was not sufficient to protect against the late response. For the antigen challenge studies in which 1E6 (anti-LFA3) was used as an isotope-matched control antibody for the HP1/2 treatment, no effect on the early or late response was observed using 1 E6 in a control trial. The 1E6-2C12 hybridoma cell line producing the 1E6 antibody has been deposited as ATCC HB 10693.

### Example 2

A subsequent experiment was performed to investigate the efficacy of aerosol delivery of the antibody. The trials were performed essentially as described above, except that two sheep were used, and the HP1/2 was delivered via nebulizer in the form of an aerosol (dose = 8 mg HP1/2 per animal, administered 1/2 hour prior to antigen challenge).

In control sheep (receiving placebo), the late phase response was characterized by an average increase in SR_{L} of 126% of the baseline value, whereas when the sheep were treated with the anti-VLA-4 antibody, average rise in SR_{L} was 26% of baseline. These results amount to approximately 80% inhibition of late phase response. The results also indicated about 70% inhibition of airway responsiveness at 24 hours. From this trial, it is apparent that inhalant delivery of the antibody may be used to obtain the benefits of this invention.

These data were confirmed and extended to 5 sheep with controls (isotype-matched 1E6 (anti-LFA3) antibody control) using a 16 mg/kg aerosol dose of HP 1/2 (n=5) or 1E6 (n=4). Figures 5 and 6 show that treatment with HP1/2 aerosol at this dose 30 minutes before antigen challenge is also effective in blocking the late response and airway hyperresponsiveness. HP1/2 aerosol treatment resulted in significant (indeed, virtually complete) inhibition of the late phase response experienced by the 1E6 controls. 1E6 aerosol treatment was without effect. Although comparable protection was achieved in both the intravenous and aerosol trials, the protection afforded by HP1/2 in the aerosol trials was achieved without detectable blood levels of the drug. This effect of HP1/2 is specific because the same dose of 1 E6 had no protective effect (e.g., 1E6 treated animals showed a significant fall in PC₄₀₀, whereas HP1/2 blocked the effect). The differences in the physiological responses between HP1/2 and 1 E6 are not the result of deficiencies in total WBC or differential counts between the groups. Total WBC and differential in both the HP 1/2 and 1 E6 groups showed a pattern of responses similar to those seen in the intravenous trials.

From the foregoing disclosure, numerous modifications and additional embodiments of the invention will be apparent to those experienced in this art. For example, actual dosage used, the type of antibody or antibody fragment used, mode of administration, exact composition, time and manner of administration of the treatment, and many other features all may be varied without departing from the description above.

### CITED PUBLICATIONS

[1] M. R. Sears, "Epidemiology of Asthma," in Asthma as an Inflammatory Disease. P. O'Byrne, ed. (Marcel Dekker, Inc.; New York 1990), pp. 15-48
[2] L. K. Altman, "Despite Gains in Treatment, Asthma Worsens," New York Times. The Doctor's World, March 26, 1991
[3] C. Starr, "Treating Asthma: A Killer Gathers Strength," Drug Topics, cover story, issue of April 8, 1991
[4] D. W. Cockcroft, "Atopy and Asthma," in Asthma as an Inflammatory Disease. P. O'Byrne, ed. (Marcel Dekker, Inc.; New York 1990), pp. 103-125
[5] F. M. C. Cuss, "The Pharmacology of Antiasthma Medications," in Asthma as an Inflammatory Disease, P. O'Byrne, ed. (Marcel Dekker, Inc.; New York 1990), pp. 199-250
[6] P. M. O'Byrne, "Airway inflammation and Asthma," in Asthma as an Inflammatory Disease, P.O'Byrne, ed. (Marcel Dekker, Inc.; New York 1990), pp. 143-157
[7] J. C. Hogg, "Pathology of Asthma," in Asthma as an Inflammatory Disease, P. O'Byrne, ed. (Marcel Dekker, Inc.; New York 1990), pp. 1-13
[8] W. M. Abraham et al., "Cellular Markers of Inflammation in the Airways of Allergic Sheep with and without Allergen-induced Late Responses," Am. Rev. Respir. Dis., 138, 1565-1571 (1988)
[9] K. F. Chung, "Inflammatory Mediators in Asthma," in Asthma as an Inflammatory Disease, P. O'Byrne, ed. (Marcel Dekker, Inc.; New York 1990), pp. 159-183
[10] P. F. Weller et al., "Human eosinophil adherence to vascular endothelium mediated by binding to vascular cell adhesion molecule 1 and endothelial leukocyte adhesion molecule 1," Proc. Natl. Acad. Sci. USA, 88, 7430-7433 (1991)
[11] G. M. Walsh et al., "Human Eosinophil, But Not Neutrophil, Adherence to IL-1-Stimulated Human Umbilical Vascular Endothelial Cells is α₄β₁ (Very Late Antigen-4) Dependent," J. Immunol., 146, 3419-3423 (1991)
[12] B. S. Bochner et al., "Adhesion of Human Basophils, Eosinophils, and Neutrophils to Interleukin 1-activated Human Vascular Endothelial Cells: Contributions of Endothelial Cell Adhesion Molecules," J. Exp. Med., 173, 1553-1556 (1991)
[13] A. Dobrina et al., "Mechanisms of Eosinophil Adherence to Cultured Vascular Endothelial Cells," J. Clin. Invest., 88, 20-26 (1991)
[14] M. J. Elices et al., "VCAM-1 on Activated Endothelium Interacts with the Leukocyte Integrin VLA-4 at a Site Distinct from the VLA-4/Fibronectin Binding Site," Cell, 60, 577-584 (1990)
[15] Kohler and Milstein, "Continuous Cultures of Fused Cells Secreting Antibody of Predefined Specificity," Nature, 256, pp. 495-7 (1975)
[16] F. Sanchez-Madrid et al., "VLA-3: A novel polypeptide association within the VLA molecular complex: cell distribution and biochemical characterization," Eur. J. Immunol., 16, 1343-1349 (1986)
[17] M. E. Hemler et al., "Characterization of the Cell Surface Heterodimer VLA-4 and Related Peptides," J. Biol. Chem., 262(24), 11478-11485 (1987)
[18] L Osborn et al., "Direct cloning of vascular cell adhesion molecule 1, a cytokine-induced endothelial protein that binds to lymphocytes," Cell. 59, 1203-1211 (1989)
[19] R. Pulido et al., "Functional Evidence for Three Distinct and Independently inhibitable Adhesion Activities Mediated by the Human Integrin VLA-4," J. Biol, Chem., 266(16), 10241-10245 (1991)
[20] W. M. Abraham, "Pharmacology of Allergen-Induced Early and Late Airway Responses and Antigen-Induced Airway Hyperresponsiveness in Allergic Sheep," Pulmonary Pharmacology, 2, pp. 33-40 (1989)
[21] P. T. Jones et al., "Replacing the Complementarity-Determining Regions in a Human Antibody with Those From a Mouse", Nature, 321, pp. 522-525 (1986)
[22] E. S. Ward et al., "Binding Activities of a Repertoire of Single Immunoglobulin Variable Domains Secreted From Escherichia coli", Nature, 341, pp. 544-546 (1989).
[23] U.S. Patent No. 4,816,397, Boss et al., "Multichain Polypeptides Or Proteins And Processes For Their Production". March 28. 1989.

## Claims

1. Use of an anti-VLA-4 antibody capable of binding to the B 1 or B2 epitope of the α4 subunit of VLA-4 for the manufacture of a medicament for use in the treatment of allergic asthma, said treatment comprising administering to a mammal suffering from allergic asthma a composition comprising an anti-VLA-4 antibody capable of binding to the B1 or B2 epitope of the α4 subunit of VLA-4; wherein said treatment does not include the use of said anti-VLA-4 antibody in combination with other antibodies having a therapeutic effect on airway responsiveness.

2. Use according to claim 1, wherein the anti-VLA-4 antibody composition is administered intravenously.

3. Use according to claim 1, wherein the anti-VLA-4 antibody composition is administered in the form of an aerosol by inhalation.

4. Use according to claim 1, 2 or 3, wherein the anti-VLA-4 antibody is selected from HP1/2, HP2/1, HP2/4, L25 and P4C2.

5. Use according to claim 1, 2 or 3 wherein the anti-VLA-4 antibody is HP1/2, or a fragment thereof capable of binding to the B1 or B2 epitope of the α4 subunit of VLA-4.

6. Use according to any preceding claim, wherein the composition is administered at a dosage so as to provide from 0.05 to 5.0 mg/kg of antibody, based on the weight of the asthma sufferer.

7. Use according to claim 6, wherein the composition is administered at a dosage so as to provide from 0.5 to 2.0 mg/kg of antibody, based on the weight of the asthma sufferer.

8. Use according to any preceding claim, wherein the composition is administered in an amount effective to provide a plasma level of antibody in the mammal of at least 10 µg/ml.

9. Use according to any preceding claim, wherein the composition is administered prior to exposure to an allergen to which said asthma sufferer is hypersensitive.

10. Use according to any of claims 1 to 8, wherein the composition is administered after exposure to an allergen to which said mammal is hypersensitive.

11. Use according to any preceding claim, wherein the mammal is human.

12. Use of a molecule capable of binding to the B1 or B2 epitope of the α4 subunit of VLA-4, the molecule being an antibody, a recombinant antibody, a chimeric antibody, or a fragment of such antibodies, or combinations of any of the foregoing molecules capable of binding to the B1 or B2 epitope of the α4 subunit of VLA-4, for the manufacture of a medicament for use in the treatment of allergic asthma, said treatment comprising administering to a mammal suffering from allergic asthma a molecule capable of binding to the B1 or B2 epitope of the α4 subunit of VLA-4, the molecule being an antibody, a recombinant antibody, a chimeric antibody, or a fragment of such antibodies, or combinations of any of the foregoing molecules capable of binding to the B1 or B2 epitope of the α4 subunit of VLA-4, in an amount effective to provide inhibition of late phase response to an allergen to which the sufferer is hypersensitive or to provide decreased airway hypersensitivity in said mammal following allergen challenge; wherein said treatment does not include the use of said molecule in combination with other antibodies having a therapeutic effect on airway hyperresponsiveness.

13. Use according to claim 12, wherein the antibody or molecule is selected from monoclonal antibody HP 1/2, Fab, Fab', F(ab')₂ or F(v) fragments of such antibody.

14. Use according to claim 12, wherein the composition comprises a plurality of anti-VLA-4 monoclonal antibodies or VLA-4-binding fragments thereof.

15. Use according to claim 12, wherein the anti-VLA-4 is HP 1/2, or a fragment thereof capable of binding to VLA-4.

16. Use according to any of claims 12 to 15, wherein the composition is administered at a dosage so as to provide from 0.05 to 5.0 mg/kg of antibody or antibody fragment, based on the weight of the asthma sufferer.

17. Use according to claim 16, wherein the composition is administered at a dosage so as to provide 1.0 - 2.0 mg/kg of antibody or antibody fragment, based on the weight of the asthma sufferer.

18. Use according to any of claims 12 to 17, wherein the composition is administered in an amount effective to provide a plasma level of antibody in the mammal of at least 10 µg/ml over a period of seven days.

19. Use of a composition comprising an anti-VLA-4 antibody HP 1/2 or a fragment thereof capable of binding to the α4 subunit of VLA-4 for the manufacture of a medicament for use in the treatment of allergic asthma, said treatment comprising administering to a mammal suffering from allergic asthma a composition comprising anti-VLA-4 antibody HP 1/2 or a fragment thereof capable of binding to the α4 subunit of VLA-4; wherein said treatment does not include the use of said anti-VLA-4 antibody in combination with other antibodies having a therapeutic effect on airway responsiveness.

20. Use of a monoclonal antibody recognizing the B1 or B2 epitope of the α4 subunit of VLA-4 for the preparation of a pharmaceutical composition for the treatment of allergic asthma, wherein said composition is effective to attenuate late phase response or significantly reduce airway hypersensitivity in an mammal suffering from allergic asthma and consists essentially of said antibody in a pharmaceutically-acceptable carrier; wherein said treatment does not include the use of said anti-VLA-4 antibody in combination with other antibodies having a therapeutic effect on airway responsiveness.

## Patentansprüche

1. Verwendung eines Anti-VLA-4-Antikörpers, der imstande ist, sich an das B1-oder B2-Epitop der α4-Untereinheit von VLA-4 anzulagern, für die Herstellung eines Medikaments zur Verwendung bei der Behandlung von allergischem Asthma, wobei die Behandlung die Verabreichung einer Zusammensetzung, umfassend einen Anti-VLA-4-Antikörper, der imstande ist, sich an das B1- oder B2-Epitop der α4-Untereinheit von VLA-4 anzulagern, an ein Säugetier, das an allergischem Asthma leidet, umfasst, wobei die Behandlung nicht die Verwendung des Anti-VLA-4-Antikörpers in Kombination mit anderen Antikörpern, die eine therapeutische Wirkung auf die Empfindlichkeit des Atemswegs haben, einschließt.

2. Verwendung gemäß Anspruch 1, wobei die Anti-VLA-4-Antikörper-Zusammensetzung intravenös verabreicht wird.

3. Verwendung gemäß Anspruch 1, wobei die Anti-VLA-4-Antikörper-Zusammensetzung durch Inhalation in der Form eines Aerosols verabreicht wird.

4. Verwendung gemäß Anspruch 1, 2 oder 3, wobei der Anti-VLA-4-Antikörper aus HP1/2, HP2/1, HP2/4, L25 und P4C2 ausgewählt wird.

5. Verwendung gemäß Anspruch 1, 2 oder 3, wobei der Anti-VLA-4-Antikörper HP1/2 oder ein Fragment davon, das imstande ist, sich an das B1- oder B2-Epitop der α4-Untereinheit von VLA-4 anzulagern, ist.

6. Verwendung gemäß einem der vorhergehenden Ansprüche, wobei die Zusammensetzung in einer derartigen Dosierung verabreicht wird, dass 0,05 bis 5,0 mg/kg Antikörper, basierend auf dem Gewicht des an Asthma Leidenden, bereitgestellt werden.

7. Verwendung gemäß Anspruch 6, wobei die Zusammensetzung in einer derartigen Dosierung verabreicht wird, dass 0,5 bis 2,0 mg/kg Antikörper, basierend auf dem Gewicht des an Asthma Leidenden, bereitgestellt werden.

8. Verwendung gemäß einem der vorhergehenden Ansprüche, wobei die Zusammensetzung in einer für die Schaffung einer Antikörperkonzentration von mindestens 10 µg/ml im Plasma des Säugetiers wirksamen Menge verabreicht wird.

9. Verwendung gemäß einem der vorhergehenden Ansprüche, wobei die Zusammensetzung vor der Einwirkung eines Allergens, gegen welches der an Asthma Leidende überempfindlich ist, verabreicht wird.

10. Verwendung gemäß einem der Ansprüche 1 bis 8, wobei die Zusammensetzung nach der Einwirkung eines Allergens, gegen welches das Säugetier überempfindlich ist, verabreicht wird.

11. Verwendung gemäß einem der vorhergehenden Ansprüche, wobei das Säugetier ein Mensch ist.

12. Verwendung eines Moleküls, das imstande ist, sich an das B1- oder B2-Epitop der α4-Untereinheit von VLA-4 anzulagern, wobei das Molekül ein Antikörper, ein rekombinanter Antikörper, ein chimärer Antikörper, ein Fragment derartiger Antikörper oder Kombinationen von beliebigen der vorhergehenden Moleküle, die imstande sind, sich an das B1- oder B2-Epitop der α4-Untereinheit von VLA-4 anzulagern, ist, für die Herstellung eines Medikaments zur Verwendung bei der Behandlung von allergischem Asthma, wobei die Behandlung die Verabreichung eines Moleküls, das imstande ist, sich an das B1- oder B2-Epitop der α4-Untereinheit von VLA-4 anzulagern, wobei das Molekül ein Antikörper, ein rekombinanter Antikörper, ein chimärer Antikörper, ein Fragment derartiger Antikörper oder Kombinationen von beliebigen der vorhergehenden Moleküle, die imstande sind, sich an das B1- oder B2-Epitop der α4-Untereinheit von VLA-4 anzulagern, ist, an ein Säugetier, das an allergischem Asthma leidet, in einer zur Schaffung der Abschwächung der Spätphasenreaktion auf ein Allergen, gegen welches der Leidende überempfindlich ist, oder zur Schaffung einer verminderten Überempfindlichkeit des Atemwegs beim Säugetier im Anschluss an eine Allergenprovokation, wirksamen Menge umfasst, wobei die Behandlung nicht die Verwendung des Moleküls in Kombination mit anderen Antikörpern, die eine therapeutische Wirkung auf die Überempfindlichkeit des Atemswegs haben, einschließt.

13. Verwendung gemäß Anspruch 12, wobei der Antikörper oder das Molekül aus dem monoklonalen Antikörper HP1/2; Fab, Fab', F(ab')₂ oder F(v)-Fragmenten eines derartigen Antikörpers ausgewählt wird.

14. Verwendung gemäß Anspruch 12, wobei die Zusammensetzung eine Vielzahl von anti-VLA-4 monoklonalen Antikörpern oder VLA-4-Bindungsfragmenten davon umfasst.

15. Verwendung gemäß Anspruch 12, wobei der Anti-VLA-4 HP1/2 oder ein Fragment davon, das imstande ist, sich an VLA-4 anzulagern, ist.

16. Verwendung gemäß einem der Ansprüche 12 bis 15, wobei die Zusammensetzung in einer derartigen Dosierung verabreicht wird, dass 0,05 bis 5,0 mg/kg Antikörper oder Antikörperfragment, basierend auf dem Gewicht des an Asthma Leidenden, bereitgestellt werden.

17. Verwendung gemäß Anspruch 16, wobei die Zusammensetzung in einer derartigen Dosierung verabreicht wird, dass 1,0 bis 2,0 mg/kg Antikörper oder Antikörperfragment, basierend auf dem Gewicht des an Asthma Leidenden, bereitgestellt werden.

18. Verwendung gemäß einem der Ansprüche 12 bis 17, wobei die Zusammensetzung in einer zur Schaffung einer Antikörperkonzentration von mindestens 10 µg/ml im Plasma des Säugetiers über einen Zeitraum von sieben Tagen wirksamen Menge verabreicht wird.

19. Verwendung einer Zusammensetzung, umfassend einen Anti-VLA-4-Antikörper HP1/2 oder ein Fragment davon, das imstande ist, sich an die α4-Untereinheit von VLA-4 anzulagern, für die Herstellung eines Medikaments zur Verwendung bei der Behandlung von allergischem Asthma, wobei die Behandlung die Verabreichung einer Zusammensetzung, umfassend den Anti-VLA-4-Antikörper HP1/2 oder ein Fragment davon, das das imstande ist, sich an die α4-Untereinheit von VLA-4 anzulagern, an ein Säugetier, das an Asthma leidet, umfasst, wobei die Behandlung nicht die Verwendung des Anti-VLA-4-Antikörpers in Kombination mit anderen Antikörpern, die eine therapeutische Wirkung auf die Empfindlichkeit des Atemswegs haben, einschließt.

20. Verwendung eines monoklonalen Antikörpers, der das B1- oder B2-Epitop der α4-Untereinheit von VLA-4 erkennt, für die Herstellung einer pharmazeutischen Zusammensetzung zur Behandlung von allergischem Asthma, wobei die Zusammensetzung wirksam ist, um die Spätphasenreaktion abzuschwächen oder die Überempfindlichkeit des Atemwegs bei einem Säugetier, das an allergischen Asthma leidet, signifikant zu vermindern, und im wesentlichen aus dem Antikörper in einem pharmazeutisch verträglichen Träger besteht, wobei die Behandlung nicht die Verwendung des Anti-VLA-4-Antikörpers in Kombination mit anderen Antikörpern, die eine therapeutische Wirkung auf die Empfindlichkeit des Atemswegs haben, einschließt.

## Revendications

1. Utilisation d'un anticorps anti-VLA-4 capable de se lier à l'épitope B1 ou B2 de la sous-unité α4 de VLA-4 pour la fabrication d'un médicament pour une utilisation dans le traitement de l'asthme allergique, ledit traitement comprenant l'administration à un mammifère souffrant d'asthme allergique d'une composition comprenant un anticorps anti-VLA-4 capable de se lier à l'épitope B1 ou B2 de la sous-unité α4 de VLA-4; dans laquelle ledit traitement n'inclut pas l'utilisation dudit anticorps anti-VLA-4 combiné avec d'autres anticorps ayant un effet thérapeutique sur la sensibilité des voies aériennes.

2. Utilisation selon la revendication 1, dans laquelle la composition à base d'anticorps anti-VLA-4 est administrée par voie intraveineuse.

3. Utilisation selon la revendication 1, dans laquelle la composition à base d'anticorps anti-VLA-4 est administrée sous la forme d'un aérosol par inhalation.

4. Utilisation selon la revendication 1, 2 ou 3, dans laquelle l'anticorps anti-VLA-4 est choisi parmi HP1/2, HP2/1, HP2/4, L25 et P4C2.

5. Utilisation selon la revendication 1, 2 ou 3, dans laquelle l'anticorps anti-VLA-4 est HP1/2 ou un fragment de celui-ci capable de se lier à l'épitope B1 ou B2 de la sous-unité α4 de VLA-4.

6. Utilisation selon l'une quelconque des revendications précédentes, dans laquelle la composition est administrée à une posologie permettant de fournir de 0,05 à 5,0 mg/kg d'anticorps, sur la base du poids de l'asthmatique.

7. Utilisation selon la revendication 6, dans laquelle la composition est administrée à une posologie permettant de fournir de 0,5 à 2,0 mg/kg d'anticorps, sur la base du poids de l'asthmatique.

8. Utilisation selon l'une quelconque des revendications précédentes, dans laquelle la composition est administrée en une quantité efficace pour fournir un taux d'anticorps dans le plasma du mammifère d'au moins 10 µg/ml.

9. Utilisation selon l'une quelconque des revendications précédentes, dans laquelle la composition est administrée avant l'exposition à un allergène auquel ledit asthmatique est hypersensible.

10. Utilisation selon l'une quelconque des revendications 1 à 8, dans laquelle la composition est administrée après exposition à un allergène auquel ledit mammifère est hypersensible.

11. Utilisation selon l'une quelconque des revendications précédentes, dans laquelle le mammifère est humain.

12. Utilisation d'une molécule capable de se lier à l'épitope B1 ou B2 de la sous-unité α4 de VLA-4, la molécule étant un anticorps, un anticorps recombinant, un anticorps chimérique, un fragment de tels anticorps, ou des combinaisons de n'importe lesquelles des molécules précédentes capables de se lier à l'épitope B1 ou B2 de la sous-unité α4 de VLA-4, pour la fabrication d'un médicament pour une utilisation dans le traitement de l'asthme allergique, ledit traitement comprenant l'administration à un mammifère souffrant d'asthme allergique d'une molécule capable de se lier à l'épitope B1 ou B2 de la sous-unité α4 de VLA-4, la molécule étant un anticorps, un anticorps recombinant, un anticorps chimérique, ou un fragment de tels anticorps, ou des combinaisons de n'importe lesquelles des molécules précédentes capables de se lier à l'épitope B1 ou B2 de la sous-unité α4 de VLA-4, en une quantité efficace pour permettre l'inhibition de la réaction en phase tardive à un allergène auquel le patient est hypersensible ou pour assurer une hypersensibilité réduite des voies aériennes chez ledit mammifère après provocation par un allergène; dans laquelle ledit traitement n'inclut pas l'utilisation de ladite molécule combinée avec d'autres anticorps ayant un effet thérapeutique sur l'hypersensibilité des voies aériennes.

13. Utilisation selon la revendication 12, dans laquelle l'anticorps ou la molécule est choisi parmi un anticorps monoclonal HP1/2, les fragments Fab, Fab', F(ab')₂ ou F(v) d'un tel anticorps.

14. Utilisation selon la revendication 12, dans laquelle la composition comprend une pluralité d'anticorps monoclonaux anti-VLA-4 ou de fragments de ceux-ci se liant au VLA-4.

15. Utilisation selon la revendication 12, dans laquelle l'anti-VLA-4 est HP1/2, ou un fragment de celui-ci capable de se lier au VLA-4.

16. Utilisation selon l'une quelconque des revendications 12 à 15, dans laquelle la composition est administrée à une posologie permettant de fournir de 0,05 à 5,0 mg/kg d'anticorps ou de fragment d'anticorps sur la base du poids de l'asthmatique.

17. Utilisation selon la revendication 16, dans laquelle la composition est administrée à une posologie permettant de fournir de 1,0 à 2,0 mg/kg d'anticorps ou de fragment d'anticorps sur la base du poids de l'asthmatique.

18. Utilisation selon l'une quelconque des revendications 12 à 17, dans laquelle la composition est administrée en une quantité efficace pour fournir un taux d'anticorps dans le plasma du mammifère d'au moins 10 µg/ml, sur une période de sept jours.

19. Utilisation d'une composition comprenant un anticorps anti-VLA-4 HP1/2 ou un fragment de celui-ci capable de se lier à la sous-unité α4 de VLA-4 pour la fabrication d'un médicament pour une utilisation dans le traitement de l'asthme allergique, ledit traitement comprenant l'administration à un mammifère souffrant d'asthme allergique d'une composition comprenant l'anticorps anti-VLA-4 HP1/2 ou un fragment de celui-ci capable de se lier à la sous-unité α4 de VLA-4; dans laquelle ledit traitement n'inclut pas l'utilisation dudit anticorps anti-VLA-4 combiné avec d'autres anticorps ayant un effet thérapeutique sur la sensibilité des voies aériennes.

20. Utilisation d'un anticorps monoclonal reconnaissant l'épitope B1 ou B2 de la sous-unité α4 de VLA-4 pour la préparation d'une composition pharmaceutique pour le traitement de l'asthme allergique, dans laquelle ladite composition est efficace pour atténuer la réaction en phase tardive ou réduire significativement l'hypersensibilité des voies aériennes chez un mammifère souffrant d'asthme allergique, et consiste essentiellement en ledit anticorps dans un véhicule pharmaceutiquement acceptable; dans laquelle ledit traitement n'inclut pas l'utilisation dudit anticorps anti-VLA-4 combiné avec d'autres anticorps ayant un effet thérapeutique sur la sensibilité des voies aériennes.
